# EUROPÄISCHE PATENTANMELDUNG

(11) **EP 2 098 169 A2**
(43) Veröffentlichungstag der Anmeldung: **09.09.2009**
(21) Anmeldenummer: 09002795.4
(22) Anmeldetag: 27.02.2009
(51) Int. Cl.: A61B 6/08, G02B 26/10

(54) **Vorrichtung und Verfahren zur Darstellung eines Feldes auf der Oberfläche eines Patientenkörpers**

(30) Priorität: 04.03.2008 DE 102008012496
(71) Anmelder: LAP GmbH Laser Applikationen, D-21337 Lüneburg (DE)
(72) Erfinder: Kindlein, Johann, 21339 Lüneburg (DE)
(74) Vertreter: Hauck Patent- und Rechtsanwälte

(57) **Zusammenfassung**

Die Erfindung betrifft eine Vorrichtung zur Darstellung eines Feldes auf der dreidimensionalen Oberfläche eines Patientenkörpers, mit einer Steuereinrichtung, die dreidimensionale Koordinaten mindestens eines auf der Oberfläche des Patientenkörpers darzustellenden Feldes bereitstellt, wobei das Feld ein Sollschnittfeld eines Strahlenfeldes auf der Oberfläche des Patientenkörpers vorgibt. Erfindungsgemäß ist vorgesehen, dass mindestens eine mindestens einen Laser aufweisende Projektionseinrichtung vorgesehen ist, mit der anhand der bereitgestellten Koordinaten das Sollschnittfeld auf die dreidimensionale Oberfläche des Patientenkörpers projizierbar ist, indem mindestens ein von dem Laser erzeugter Laserstrahl ausreichend schnell entlang der Kontur des Sollschnittfeldes geführt werden kann, so dass der Eindruck einer geschlossenen Kontur um das Sollschnittfeld entsteht. Die Erfindung betrifft außerdem ein entsprechendes Verfahren.

## Beschreibung

Die Erfindung betrifft eine Vorrichtung und ein Verfahren zur Darstellung eines Feldes auf der Oberfläche eines Patientenkörpers und bezieht sich auf das Gebiet der Strahlungstherapie mit ionisierender Strahlung zur Krebsbehandlung. Dabei werden üblicherweise mehrere Strahlen aus unterschiedlichen Richtungen auf den zu behandelnden Körper gerichtet, so dass sie sich in einem Isozentrum schneiden. Dort wirkt die summierte Strahlendosis der verschiedenen auf das Isozentrum gerichteten ionisierenden Strahlen. Auf diese Weise wird die Beeinträchtigung des umliegenden Gewebes minimiert.

Als erster Schritt einer Strahlentherapie wird üblicherweise eine Computertomographie-Aufnahme (CT) des auf einer Lagerungshilfe, wie einem Behandlungstisch, gelagerten und fixierten Patienten erstellt. Anhand dieser Aufnahme wird ein 3D-Modell des Patienten erstellt und der zu behandelnde Tumor lokalisiert und ein Bestrahlungsplan erstellt. Dies umfasst die Konturierung der Zielvolumina sowie die Berechnung der Dosis für die Bestrahlung und dabei insbesondere die Bestimmung der Anzahl und Lage der Strahlenfelder des Bestrahlungsgeräts, so dass der Tumor wie gewünscht bestrahlt wird.

Während der Durchführung dieser Bestrahlungsplanung befindet sich der Patient nicht mehr auf der Lagerungshilfe. Außerdem findet im Rahmen einer solchen Strahlentherapie üblicherweise eine Vielzahl von Bestrahlungsterminen (üblicherweise bis zu 30 Fraktionen) statt. Daher ist wiederholt eine Übertragung der auf Grundlage des erstellten 3D-Modells des Patienten berechneten Strahlenfelder auf den realen Patienten erforderlich. Dieser Schritt wird auch als "Simulation" bezeichnet. Insbesondere muss der Patient für die Bestrahlung reproduzierbar so auf den Behandlungstisch gelegt werden, wie er bei der Erstellung der CT-Aufnahme lag.

Dazu ist es bekannt, eine Positionierung des Patienten mittels 3D-Röntgenaufnahmen ("Cone Beam") durchzuführen, wobei beispielsweise Knochen des Patienten die Referenzpunkte für eine Ausrichtung des Patienten bilden. Der Vorteil dieser Vorgehensweise ist eine hohe Genauigkeit. Außerdem müssen keine Referenzmarkierungen oder ähnliches auf der Patientenhaut aufgebracht werden. Der Nachteil dieser Vorgehensweise ist eine hohe Röntgenstrahlungsdosis für den Patienten, da die Röntgenpositionierung vor jeder Behandlungsfraktion erforderlich ist. Im Rahmen einer Strahlentherapie kann dies also üblicherweise bis zu 30 Mal erfolgen. Diese in einem großen Volumen auf den Körper applizierte Strahlendosis kann insbesondere bei jüngeren Patienten langfristig wieder einen neuen Krebs induzieren.

Gemäß einer bekannten alternativen Vorgehensweise kann der Patient anhand von drei Referenzpunkten auf seiner Haut, gebildet beispielsweise durch entsprechende Retroreflektoren, auf der Lagerungshilfe positioniert werden. Ein solches Verfahren ist beispielsweise bekannt aus DE 44 18 216 A1. Nach Durchführung der Bestrahlungsplanung werden die Schnittpunkte der Strahlenfelder mit der Hautoberfläche berechnet, die für die gewünschte Bestrahlung erforderlich sind. Die Schnittpunkte liegen dann in Form einer 3D-Koordinatentabelle vor. Nach der Neupositionierung des Patienten anhand der drei Referenzmarkierungen können dann die Koordinaten aus der Tabelle mit einem Lasersystem nacheinander auf der Körperoberfläche des Patienten dargestellt werden. Das Anfahren der Koordinaten kann beispielsweise durch eine Infrarot-Fernbedienung von einer Bedienperson gesteuert werden. Die von dem Laser jeweils dargestellten Punkte werden dann manuell, beispielsweise mit einem Stift, auf der Haut des Patienten eingezeichnet. Durch eine Verbindung der Punkte ergibt sich das gewünschte Schnittfeld für die Bestrahlung.

Ein solches Verfahren ist beispielsweise bekannt aus DE 44 21 315 A1 oder DE 195 24 951 A1. Die dazu eingesetzte Lasereinrichtung besteht insbesondere aus fünf motorisch beweglichen Lasern, wobei zwei in der Höhe verstellbare Laser eine horizontale Linie entlang des Behandlungstisches auf den Patienten richten, jeweils rechts und links von dem Behandlungstisch. Die übrigen drei Laser sind in einer Platte montiert, die sich über dem Behandlungstisch, beispielsweise einem CT-Tisch, befindet. Dabei ist ein Laser quer zur Tisch-Längsrichtung verschiebbar und richtet eine Linie entlang des Tisches auf den Patienten. Die beiden übrigen Laser in der Platte sind miteinander gekoppelt und richten eine gemeinsame Linie quer zur Längsachse des Tisches auf den Patienten. Durch die Kopplung zweier Laser können auch solche Koordinaten auf der Haut des Patienten dargestellt werden, die ansonsten unterhalb des Querdurchmessers des Patienten verschattet würden. Mit dem beschriebenen System ist es möglich, nahezu beliebige Koordinaten auf der Haut des Patienten anzufahren, wobei eine Koordinate jeweils durch ein Kreuz zweier Laserlinien angezeigt wird. Ein solches Lasersystem wird von der Anmelderin unter dem Namen "Dorado CT4" angeboten.

Die beschriebene Vorgehensweise ist allerdings verhältnismäßig zeitaufwendig und damit teuer, da Nutzungszeit in dem CT-Raum sehr teuer ist. Darüber hinaus ist das manuelle Einzeichnen der Punkte, insbesondere abhängig von der Adiposität des Patienten nicht immer ausreichend genau. Insbesondere können sich Hautmarkierungen verschieben. Daher finden in der Praxis nur teilweise Markierungen der Schnittfelder auf der Haut statt. Entsprechend ist die korrekte Bestrahlung des Patienten nicht immer ausreichend genau gewährleistet.

Die Simulation ist nicht nur eine Übertragung des Bestrahlungsplans auf den Patienten, sondern auch ein wichtiges Element im Rahmen der Qualitätssicherung. Zum letzten Mal vor der Therapie wird der Bestrahlungsplan auf seine Plausibilität geprüft und festgestellt, ob die Strahlenfelder reproduzierbar auf das gewünschte Planungszielvolumen eingestellt werden können.

Ausgehend von dem erläuterten Stand der Technik liegt der Erfindung die Aufgabe zugrunde, eine Vorrichtung und ein Verfahren der eingangs genannten Art bereitzustellen, mit denen der Simulationsprozess der Strahlentherapie in einfacher, schneller und präziser Weise und dabei ohne Gefahr für die Gesundheit des Patienten möglich ist.

Diese Aufgabe wird gemäß der Erfindung durch die Gegenstände der unabhängigen Ansprüche 1 und 16 gelöst. Vorteilhafte Ausgestaltungen finden sich in den abhängigen Ansprüchen sowie der Beschreibung und den Figuren. Für eine Vorrichtung zur Darstellung eines Feldes auf der dreidimensionalen Oberfläche eines Körpers, insbesondere eines Patientenkörpers, mit einer Steuereinrichtung, die dreidimensionale Koordinaten mindestens eines auf der Oberfläche des Patientenkörpers darzustellenden Feldes bereitstellt, wobei das Feld ein Sollschnittfeld eines Strahlenfeldes auf der Oberfläche des Patientenkörpers vorgibt, wird die Aufgabe gemäß der Erfindung dadurch gelöst, dass mindestens eine mindestens einen Laser aufweisende Projektionseinrichtung vorgesehen ist, mit der anhand der bereitgestellten Koordinaten das Sollschnittfeld auf die dreidimensionale Oberfläche des Patientenkörpers projizierbar ist, indem mindestens ein von dem Laser erzeugter Laserstrahl ausreichend schnell entlang der Kontur des Sollschnittfelds geführt werden kann, so dass der Eindruck einer geschlossenen Kontur um das Sollschnittfeld entsteht.

Für ein Verfahren zur Darstellung eines Feldes auf der dreidimensionalen Oberfläche eines Körpers, insbesondere eines Patientenkörpers, bei dem von einer Steuereinrichtung dreidimensionale Koordinaten mindestens eines auf der Oberfläche des Patientenkörpers darzustellenden Feldes bereitgestellt werden, wobei das Feld ein Sollschnittfeld eines Strahlenfeldes auf der Oberfläche des Patientenkörpers vorgibt, wird die Aufgabe gemäß der Erfindung dadurch gelöst, dass mit mindestens einer mindestens einen Laser aufweisenden Projektionseinrichtung anhand der bereitgestellten Koordinaten das Sollschnittfeld auf die dreidimensionale Oberfläche des Patientenkörpers projiziert wird, indem mindestens ein von dem Laser erzeugter Laserstrahl ausreichend schnell entlang der Kontur des Sollschnittfelds geführt wird, so dass der Eindruck einer geschlossenen Kontur um das Sollschnittfeld entsteht. Die Steuereinrichtung der Projektionseinrichtung stellt auch die dreidimensionalen Koordinaten der Oberfläche des Patientenkörpers bereit. Das darzustellende Feld (Sollschnittfeld) ist dabei so gewählt, dass die vorgesehene Körperregion von der Strahlung in der im Rahmen der Bestrahlungsplanung gewünschten und berechneten Weise bestrahlt wird. Das Feld ist dabei abhängig von der Form eines Mulit-Leaf-Kollimators eines Bestrahlungsgeräts, beispielsweise eines Linearbeschleunigers, und/oder der Position des Isozentrums eines zu behandelnden Tumors. Dabei können insbesondere mehrere Felder dargestellt werden, sofern mehrere Strahlen für die Bestrahlung eingesetzt werden, die sich in einem in dem Körper des Patienten lokalisierten Isozentrum schneiden sollen. Die Grundidee der Erfindung ist dabei, mittels eines Laserprojektors als optisches System die von der Steuereinrichtung bereitgestellten, im Rahmen der Bestrahlungsplanung ermittelten 3D-Koordianten für das Sollschnittfeld nacheinander anzufahren. Dabei werden die Koordinatenpunkte von dem Laserstrahl so schnell angefahren, dass für einen menschlichen Betrachter eine geschlossene Kontur bzw. ein geschlossener Umriss um das Sollschnittfeld auf der Haut erscheint. Es wird also ein direkter Zusammenhang zwischen dem virtuellen Patienten im Computer, z.B. auf Grundlage eines CT-Bildes, und dem realen Patienten auf dem CT-Tisch hergestellt. Der Vorteil dieser "virtuellen Simulation" ist eine besonders schnelle und einfache Darstellung der gewünschten Strahlenfelder auf der Haut, bzw. eine entsprechend schnell und einfach mögliche Markierung des Patienten, beispielsweise mit einem Stift, auf Grundlage der Laserprojektion. Insbesondere ist kein zeitaufwendiges manuelles Anfahren von Koordinaten aus der Tabelle der Bestrahlungsplanung erforderlich. Vielmehr werden diese Koordinaten automatisch von dem Laserprojektor angefahren und so das Feld auf die Körperoberfläche projiziert. Der Einsatz gesundheitsgefährdender Röntgenstrahlung ist nicht erforderlich.

Die erfindungsgemäße Vorrichtung kann in dem Bestrahlungsraum mit dem Bestrahlungsgerät, beispielsweise einem Linearbeschleuniger ("LINAC"), in dem CT-Raum mit einem CT-Gerät oder in einem separaten Raum angeordnet sein. Entsprechend kann zu der Vorrichtung auch ein Bestrahlungsgerät oder ein CT vorgesehen sein. Insbesondere können die von der Steuereinrichtung bereitgestellten Koordinaten des Sollschnittfelds auf Grundlage einer CT-Aufnahme des Patienten erstellt werden. Darüber hinaus kann die Vorrichtung ein Computersystem mit einer graphischen Bilddarstellung und Software (Algorithmen) zur virtuellen Simulation einer Bestrahlung eines Patienten auf Grundlage von CT-Bildern mit Schnittstellen zur Übertragung von Bilddaten, Bestrahlungsdaten, Bestrahlungsfeldkonturen usw. aufweisen. Dieses Computersystem kann Teil der Steuereinrichtung sein. Die verschiedenen Einrichtungen der Vorrichtung können über ein lokales Netzwerk verbunden werden. Der Austausch der Daten kann in besonders einfacher Weise über den Digital Imaging and Communications in Medicine (DICOM und DICOM RT) Standard erfolgen. So kann das System unterschiedliche Funktionalitäten und Rechte in unterschiedlichen Räumen vergeben. Sämtliche Dateien können zur Qualitätskontrolle auf einer Server-Datei abgelegt werden.

Auch gemäß der Erfindung ist eine Markierung des Patienten mit einem Stift oder ähnlichem entlang des projizierten Umrisses möglich, jedoch nicht zwingend erforderlich. Allerdings kann auf Grundlage der Projektion das dargestellte Sollschnittfeld auf der Haut in besonders einfacher Weise markiert, beispielsweise mit einem Stift oder ähnlichem eingezeichnet werden. Die Vorrichtung muss dann nicht in dem Bestrahlungsraum angeordnet sein. Stattdessen kann die Simulation in einem separaten Raum erfolgen, dessen Benutzung kostengünstiger ist als die des Bestrahlungs- oder CT-Raums. Außerdem muss dann der Bestrahlungs- oder CT-Raum nicht durch den Einbau der erfindungsgemäßen Vorrichtung umgerüstet werden. Nach der Markierung des Patienten kann in dem Bestrahlungsraum mit dem Bestrahlungsgerät das das Behandlungsfeld simulierende Lichtfeld auf den Patienten gerichtet werden und der Patient so ausgerichtet werden, dass die Markierung und das Lichtfeld deckungsgleich sind. Dies kann manuell oder automatisch geschehen, wie weiter unten näher erläutert werden wird.

In an sich bekannter Weise kann die Ausrichtung des Patienten vor der Projektion des Feldes anhand von beispielsweise drei Referenzmarkierungen erfolgen, die z.B. von einem Laser erkannt werden und anschließend der Patient insbesondere über eine Ansteuerung der Lagerungshilfe ausgerichtet wird, wie dies beispielsweise aus DE 44 18 216 A1 bekannt ist.

In besonders einfacher Weise kann die Projektion erfolgen, wenn die Projektionseinrichtung mindestens zwei drehbare Spiegel aufweist, mit denen der Laserstrahl auf die Oberfläche des Patientenkörpers reflektiert und entlang der Kontur des Sollschnittfelds geführt werden kann. Die Spiegel können beispielsweise elektrisch angetriebene Galvanometerspiegel sein. Mit dieser Ausgestaltung wird eine besonders hohe Präzision bei der Projektion erreicht. Damit das System funktionsfähig ist, muss eine Kalibrierung der Projektionseinrichtung durchgeführt werden, indem Kalibrierkoordinaten zur Verfügung gestellt werden, die mit der Steuereinrichtung angesteuert werden und somit die Kalibrierparameter bestimmt werden. Es müssen wenigstens sechs Punkte angesteuert werden. Einer der Kalibrierpunkte, der zur Kalibrierung verwendet wird, liegt dabei im Isozentrum einer CT-Anlage oder eines Linearbeschleunigers.

Die Vorrichtung kann ein Bestrahlungsgerät zum Erzeugen ionisierender Strahlung für die Strahlentherapie aufweisen, wobei mit dem Bestrahlungsgerät eine der von dem Bestrahlungsgerät erzeugten Strahlung entsprechende Lichtstrahlung erzeugbar ist und, wobei die Vorrichtung eine Sensorvorrichtung aufweist, mit der das Sollschnittfeld und ein Lichtschnittfeld der Lichtstrahlung des Bestrahlungsgeräts auf der Oberfläche des Patientenkörpers aufgenommen werden können. Mit dieser Ausgestaltung ist eine manuelle Markierung des Patienten, beispielsweise mit einem Stift, nicht unbedingt erforderlich. Insbesondere ist die Vorrichtung dann in demselben Raum angeordnet wie das für die Bestrahlung genutzte Bestrahlungsgerät. Das Bestrahlungsgerät dient zur Tumorbehandlung und kann beispielsweise ein Linearbeschleuniger sein. Die Sensoreinrichtung kann beispielsweise eine Kamera sein. Für das Sollschnittfeld und das Lichtschnittfeld können dabei unterschiedliche Koordinatensysteme gelten. Das Lichtschnittfeld wird insbesondere durch sichtbares Licht dargestellt und simuliert das Behandlungsfeld. Dabei kann eine Auswerteeinrichtung vorgesehen sein, die dazu ausgebildet ist, eine Abweichung zwischen dem Sollschnittfeld und dem Lichtschnittfeld zu ermitteln. Die Auswerteeinrichtung kann beispielsweise in die Steuereinrichtung integriert sein. Sie kann eine Bildauswertungssoftware zum 3D-Abgleich bzw. Überlappen ("Matching") des Sollschnittfelds mit dem Lichtschnittfeld umfassen.

Die erfindungsgemäße Vorrichtung kann eine den Patientenkörper aufnehmende, mittels geeigneter Verstellantriebe entlang mindestens dreier Freiheitsgrade verstellbar gelagerte Lagerungshilfe, insbesondere einen Behandlungstisch, aufweisen. Die Freiheitsgrade können insbesondere drei orthogonal zueinander ausgerichtete Achsen sein. Eine Verstellbarkeit des Tisches kann auch entlang mehr als drei Freiheitsgraden, beispielsweise nach 6 Freiheitsgraden möglich sein, so dass beispielsweise eine Rotation der Lagerungshilfe möglich ist. Die Lagerungshilfe verfügt somit über ausreichend Freiheitsgrade, damit der zu behandelnde Patient in die erforderliche Lage gebracht werden kann, die er bereits bei der Bestrahlungsplanung, insbesondere der Erstellung des CT-Bildes zur Bildung eines 3D-Modells des Patienten, hatte.

Die erfindungsgemäße Auswerteeinrichtung kann dazu ausgebildet sein, eine für eine Deckungsgleichheit zwischen dem Sollschnittfeld und dem Lichtschnittfeld erforderliche Position der Lagerungshilfe zu ermitteln. Sie kann weiterhin dazu ausgebildet sein, die Verstellantriebe der Lagerungshilfe so anzusteuern, dass die Lagerungshilfe in die erforderliche Position bewegt wird. Ein Matching der von der Sensoreinrichtung aufgenommenen Felder liegt dabei dann und nur dann vor, wenn sich der Patient in derselben Lage befindet wie bei der Bestrahlungsplanung. Die Auswerteeinrichtung verfügt über einen entsprechenden Auswertealgorithmus, um die beiden 3D-Konturen durch eine Verlagerung der Lagerungshilfe in Überlappung zu bringen. Die Parameter zur Verstellung der Lagerungshilfe können dabei automatisch oder auf Eingabe einer Bedienperson auf die Verstellantriebe übertragen werden.

Selbstverständlich können die beschriebene Überlappung der 3D-Konturen und die anschließende Positionierung der Lagerungshilfe in analoger Weise auch anhand eines Vergleichs zwischen einem manuell auf den Patienten eingezeichneten Sollschnittfeld, beispielsweise einer Stiftmarkierung, und dem Lichtschnittfeld des Bestrahlungsgeräts erfolgen. Eine Projektion des Sollschnittfelds in dem Bestrahlungsraum ist dann nicht erforderlich.

Gemäß einer weiteren Ausgestaltung der Erfindung kann eine Einrichtung zur Ermittlung der Topographie eines auf der Lagerungshilfe befmdlichen Patientenkörpers vorgesehen sein, die mindestens einen Laserscanner aufweist, mit dem eine Laserlinie auf den Patientenkörper gerichtet werden kann, und mindestens eine Aufnahmeeinrichtung, mit der die von dem Patientenkörper reflektierte Laserlinie aufgenommen werden kann. Ein solches Laserscanning-System wird von der Anmelderin beispielsweise unter dem Namen "Galaxy" angeboten. Die Laserlinie kann dabei mit dem Laserscanner sukzessive über den Patientenkörper geführt werden, wobei die Aufnahmeeinrichtung, beispielsweise ein entsprechender Sensor (Matrixsensor), die von dem Patientenkörper reflektierten Laserlinien sukzessive aufnimmt. Aus den Sensordaten kann dann die Topographie des gesamten Körpers oder von Teilen eines Patientenkörpers ermittelt werden. Die Wellenlänge des Laserscanning-Systems kann unterschiedlich zu der Wellenlänge der Projektionseinrichtung, die das Sollschnittfeld auf die Oberfläche projiziert, gewählt werden, um unerwünschte Beeinflussungen zu vermeiden. Weiterhin kann eine Topographieauswerteeinrichtung vorgesehen sein, die dazu ausgebildet ist, aus den von der Aufnahmeeinrichtung aufgenommenen Daten die Topographie des Patientenkörpers zu ermitteln, mit einer Solltopographie zu vergleichen und anhand des Vergleichs eine für eine Deckungsgleichheit zwischen der gemessenen Topographie und der Solltopographie der Lagerungshilfe zu ermitteln. Die Solltopographie kann beispielsweise aus einer im Rahmen der Bestrahlungsplanung erstellten CT-Aufnahme oder aufgenommenen Referenztopographie stammen. Die Topographieauswerteeinrichtung kann dazu ausgebildet sein, die Lagerungshilfe bzw. die Verstellantriebe der Lagerungshilfe so zu bewegen bzw. anzusteuern, dass die Lagerungshilfe in die erforderliche Position bewegt wird. Mit dieser Ausgestaltung ist eine besonders präzise Positionierung des Patienten möglich, ohne dass die Positionierung auf Grundlage von durch Retroreflektoren o.ä. gebildeten Referenzpunkten erfolgen muss. Darüber hinaus kann bei dieser Ausgestaltung die Projektion des Sollschnittfelds auf den Patientenkörper in einem von dem Behandlungs- und/oder CT-Raum separaten Raum erfolgen, wobei dennoch eine präzise und zuverlässige Positionierung des Patienten und damit Projektion des Sollschnittfelds erreicht wird und visuell vom medizinischen Personal überprüft werden kann.

Erfindungsgemäß können insbesondere mehrere Laserprojektionseinrichtungen, beispielsweise zwei, vorgesehen sein, um an sämtlichen Positionen des Patientenkörpers das Sollschnittfeld darstellen zu können. So kann beispielsweise jeweils mindestens ein links und rechts der den Patienten aufnehmenden Lagerungshilfe oberhalb der Lagerungshilfe angeordneter Projektor vorgesehen sein. Es ist jedoch auch möglich, dass die Projektionseinrichtung entlang einer im Wesentlichen kreisförmigen Bahn um eine den Patienten aufnehmende Lagerungshilfe bewegbar angeordnet ist. Insbesondere ist dann ein Laserprojektor ausreichend, der auf einer kreisförmigen Schiene oberhalb und/oder unterhalb der Lagerungshilfe verfahren werden kann, so dass er sämtliche Körperregionen eines Patienten erreichen und das Feld auf diesen darstellen kann. Die zur Darstellung eines berechneten Feldes erforderlichen jeweiligen Haltepositionen des Projektors auf der Schiene können beispielsweise von der Steuereinrichtung oder dem jeweils eingesetzten virtuellen Simulationsprogramm bestimmt und eine Antriebseinrichtung zum Verfahren der Projektionseinrichtung entsprechend angesteuert werden.

Gemäß einer weiteren Ausgestaltung der Erfindung kann eine Einrichtung zur Echtzeit- oder Nah-Echtzeitermittlung einer dreidimensionalen Oberflächenform eines auf einer Lagerungshilfe befindlichen Patientenkörpers vorgesehen sein, wobei von der Einrichtung ermittelte dreidimensionale Oberflächendaten in Echtzeit oder Nah-Echtzeit an die Steuereinrichtung übermittelbar sind. Für die Oberflächenerfassung in Echtzeit oder Nah-Echtzeit sind entsprechende Systeme vorgesehen. Durch die Atmung des Patienten verändert sich dessen Oberfläche, insbesondere im Bereich des Thorax. Dies kann zu unerwünschten Positionsabweichungen der Bestrahlungsfelder führen. Gemäß dieser Ausgestaltung der Erfindung kennt die Steuereinrichtung und damit der Laserprojektor, der das Sollschnittfeld auf die Körperoberfläche projiziert, die jeweilige Oberfläche des Patienten in Echtzeit oder Nah-Echtzeit, also auch bei einer Veränderung aufgrund beispielsweise einer Atmungsaktivität. Die Einrichtung zur Echtzeit- oder Nah-Echtzeitermittlung der Oberflächenform des Patientenkörpers kann mindestens einen Laser aufweisen, mit dem mindestens eine Laserlinie auf den Patientenkörper gerichtet werden kann, und mindestens einen Matrixsensor, mit dem die von dem Patientenkörper reflektierte Laserlinie detektiert werden kann. Der Matrixsensor ist ein Sensor mit einer Mehrzahl von insbesondere parallel angeordneten Sensorzeilen. Diese können insbesondere senkrecht zu der bzw. den auf der Patientenoberfläche abgebildeten Laserlinie(n) verlaufen. Bei einer Veränderung der Oberfläche des Patienten resultiert eine entsprechende Veränderung des Sensorsignals. Daraus ist die Ermittlung der Oberflächenform, beispielsweise im Bereich des Thorax, möglich. Eine Ausgestaltung zur Realisierung dieses Verfahrens ist beispielsweise bekannt aus US 6,088,106 A. Die Wellenlänge der Laserlinie des Oberflächenerfassungssystems kann zur Vermeidung von unerwünschten Beeinflussungen unterschiedlich gewählt werden zu der Wellenlänge der Projektionseinrichtung, die das Sollschnittfeld auf die Oberfläche projiziert (und gegebenenfalls auch anders als die Wellenlänge eines Laserscanning-Systems).

Auf Grundlage der Echtzeit- oder Nah-Echtzeitoberflächenerfassung kann die Steuereinrichtung dazu ausgebildet sein, die Projektionseinrichtung so anzusteuern, dass diese das Sollschnittfeld nur bei einer vorgegebenen Oberflächenposition des Patientenkörpers auf die dreidimensionale Oberfläche des Patientenkörpers projiziert. Insbesondere ist dabei eine Steuerung entsprechend dem Atmungszyklus des Patienten möglich. So wird nur bei einer bestimmten Position der Patientenoberfläche, beispielsweise einer bestimmten Atmungsposition, das Feld auf den Körper projiziert. Es findet somit eine gesteuerte ("gating") Projektion statt. Auf diese Weise wird sichergestellt, dass es durch die Atmung des Patienten nicht zu einer Verfälschung des Ergebnisses durch eine unerwünschte Abweichung des projizierten Feldes von dem Sollschnittfeld kommt.

Es ist auch möglich, die Steuereinrichtung dazu auszubilden, die der Projektionseinrichtung zur Verfügung gestellten Koordinaten der Oberfläche des Patientenkörpers und des Sollschnittfelds in Abhängigkeit von der jeweiligen Oberfläche des Patientenkörpers in Echtzeit oder Nah-Echtzeit zu erfassen Bei dieser Ausgestaltung findet also eine Neuerfassung und Neuberechnung der 3D-Koordinaten in Echtzeit oder Nah-Echtzeit statt, so dass bei jeder Oberflächenform das gewünschte Sollschnittfeld projiziert wird.

Die erfindungsgemäße Vorrichtung kann insbesondere zur Durchführung des erfindungsgemäßen Verfahrens geeignet sein. Entsprechend kann das erfindungsgemäße Verfahren mit der erfindungsgemäßen Vorrichtung durchgeführt werden.

Ein Ausführungsbeispiel der Erfindung wird nachfolgend anhand einer Zeichnung näher erläutert. Es zeigen schematisch:
- Fig. 1: eine erfindungsgemäße Vorrichtung gemäß einer ersten Ausgestal- tung,
- Fig. 2: eine erfindungsgemäße Vorrichtung gemäß einer zweiten Ausgestal- tung,
- Fig. 3: eine erfindungsgemäße Vorrichtung gemäß einer dritten Ausgestal- tung, und
- Fig. 4: eine schematische Darstellung zur Veranschaulichung der Funktions- weise der erfindungsgemäßen Vorrichtung.

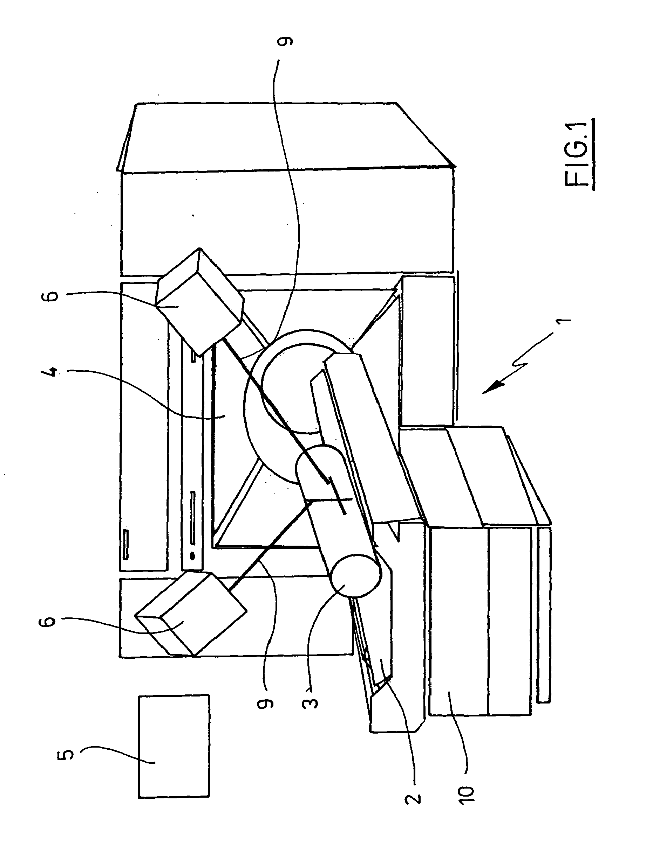

Soweit nichts anderes bestimmt ist, bezeichnen in den Figuren gleiche Bezugszeichen gleiche Gegenstände. In Fig. 1 ist eine erfindungsgemäße Vorrichtung 1 zur Darstellung eines Feldes auf der Oberfläche eines Patientenkörpers dargestellt. Die Vorrichtung weist eine mittels nicht näher dargestellter Verstellantriebe entlang mindestens dreier Achsen verstellbar gelagerte Lagerungshilfe 2, in dem Beispiel einen CT-Tisch 2, auf. Auf dem CT-Tisch 2 ist äußert schematisch ein Körper 3, beispielsweise ein Patientenkörper 3, dargestellt. Die Vorrichtung 1 umfasst weiterhin ein in einem Gehäuse angeordnetes Computertomographiegerät 4. In diesem CT-Gerät 4 können in an sich bekannter Weise CT-Aufnahmen und auf dieser Grundlage ein dreidimensionales Schnittbild eines Patienten erstellt werden. Anschließend findet im Rahmen einer Bestrahlungsplanung eine Ermittlung der Zielvolumina für die Bestrahlung mit ionisierender Strahlung statt. In einer in eine Steuereinrichtung 5 integrierte Auswerteeinrichtung werden auf Grundlage anhand des 3D-Schnittbildes des Patienten erstellter Zielvolumina die Koordinaten eines entsprechenden Sollschnittfelds eines für die Strahlentherapie vorgesehenen Therapiefeldes auf der Oberfläche des Patientenkörpers 3 berechnet. Die Steuereinrichtung 5 stellt diese 3D-Koordinaten anschließend Projektionseinrichtungen (Laserprojektoren) zur Verfügung.

Die in Fig. 1 dargestellte Vorrichtung umfasst zwei jeweils einen Laser aufweisende Projektionseinrichtungen 6. Über nicht näher dargestellte Verbindungsleitungen werden die Koordinaten von der Steuereinrichtung 5 mindestens einer der Projektionseinrichtungen 6 zugeführt. Die Projektionseinrichtungen 6 sind dabei ortsfest in dem die Vorrichtung aufnehmenden Raum angeordnet und dabei unter einem solchen Winkel zu dem Patientenkörper vorgesehen, dass sämtliche Regionen des Patientenkörpers 3 durch die Laser der Projektionseinrichtungen 6 gemeinsam erreicht werden können. Die Koordinaten werden dabei von der Steuereinrichtung 5 an diejenige Projektionseinrichtung 6 geleitet, die die jeweils gewünschte Körperregion des Patienten 3 abdeckt. Anhand der bereitgestellten Koordinaten wird dann von der Projektionseinrichtung 6 das Sollschnittfeld auf die Oberfläche des Patientenkörpers 3 projiziert, indem ein von dem Laser erzeugter Laserstrahl 9 ausreichend schnell entlang der Kontur des Sollschnittfelds geführt wird, so dass für einen menschlichen Beobachter der Eindruck einer geschlossenen Kontur um das Sollschnittfeld entsteht. Der Laserstrahl 9 wird über zwei elektrisch angetriebene Galvanometerspiegel entlang der Kontur des Sollschnittfelds geführt. Anschließend kann das Sollschnittfeld auf der Haut beispielsweise manuell markiert werden, z.B. mit einem Stift oder ähnlichem.

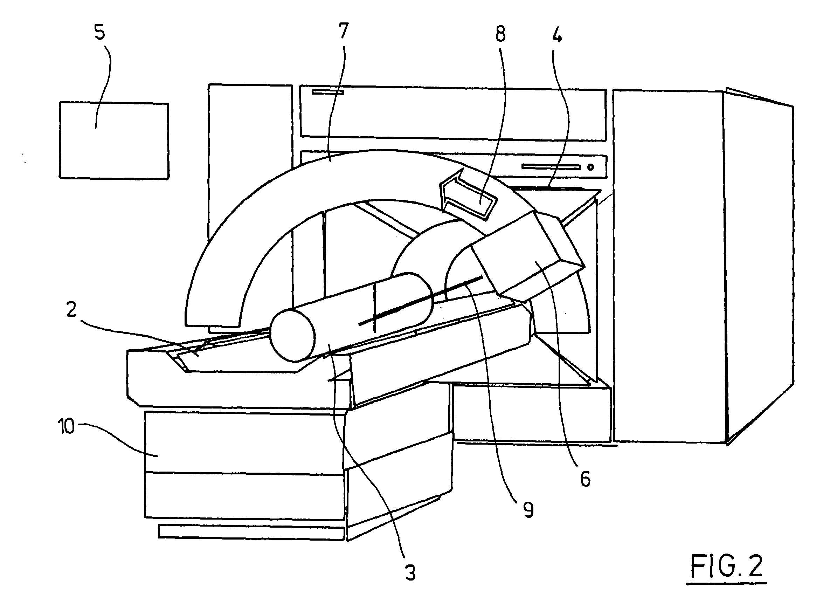

In Fig. 2 ist eine weitere Ausgestaltung der erfindungsgemäßen Vorrichtung dargestellt. Diese unterscheidet sich von der Vorrichtung aus Fig. 1 lediglich dadurch, dass anstelle zweier ortsfest angeordneter Projektionseinrichtungen 6 nur eine Projektionseinrichtung 6 vorgesehen ist, die mittels nicht näher dargestellter Verstellantriebe entlang einer oberhalb des Behandlungstisches 2 verlaufenden kreisförmigen Schiene 7 verfahren werden kann, wie durch den Pfeil 8 schematisch angedeutet. Die jeweilige Halteposition der Projektionseinrichtung 6 entlang der Schiene 7 kann dabei durch die Steuereinrichtung 5 in Abhängigkeit von den Koordinaten des jeweils darzustellenden Sollschnittfelds gewählt und die Verstellantriebe entsprechend angesteuert werden. Mit dieser Ausgestaltung ist nur eine Projektionseinrichtung 6 erforderlich, um auf der gesamten Oberfläche des Patientenkörpers 3 das Sollschnittfeld darstellen zu können.

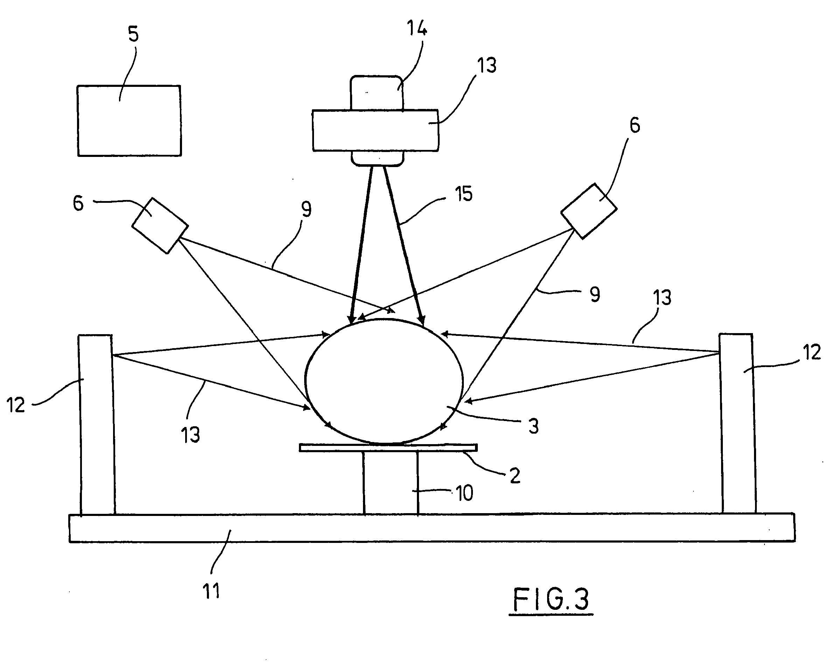

In Fig. 3 ist eine erfindungsgemäße Vorrichtung gemäß einer weiteren Ausgestaltung dargestellt. Gemäß dieser Ausgestaltung sind zwei ortsfest angeordnete Projektionseinrichtungen 6 zusammen mit der Steuereinrichtung 5 in dem für die Strahlentherapie genutzten Bestrahlungsraum angeordnet. Wiederum ist ein Patientenkörper 3 äußerst schematisch auf einer Lagerungshilfe 2 gezeigt, die über einen Sockel 10 auf dem Boden 11 des Behandlungsraums abgestützt ist. Die Vorrichtung in Fig. 3 weist weiterhin zwei ortsfest und auf beiden Seiten des Patientenkörpers 3 gegenüberliegend angeordnete Laser 12 auf. Diese dienen in an sich bekannter Weise zu einer Positionierung des Patientenkörpers 3 auf der Lagerungshilfe 2 anhand von an dem Patientenkörper 3 angeordneten Markierungen. Anschließend erfolgt eine Ansteuerung der Lagerungshilfe 2 so, dass der Patientenkörper 3 in die vorgegebene, während der Bestrahlungsplanung eingenommene Position bewegt wird.

Alternativ oder zusätzlich zu den Lasern 12 kann auch ein in Fig. 3 lediglich schematisch dargestelltes Laserscanning-System 13 oder ein anderes System zur Erfassung der Körperoberfläche vorgesehen sein. Ein solches, von der Anmelderin beispielsweise unter dem Namen "Galaxy" angebotenes System weist mindestens einen Laserscanner auf, mit dem eine nicht näher dargestellte Laserlinie auf den Patientenkörper 3 gerichtet werden kann. Weiterhin weist das System 13 eine in das System 13 integrierte Aufnahmeeinrichtung auf, mit der die von dem Patientenkörper reflektierte Laserlinie aufgenommen werden kann. Die Laserlinie wird von dem System 13 sukzessive über den Patientenkörper 3 geführt und dabei jeweils von der Aufnahmeeinrichtung das reflektierte Laserlicht gemessen. Mittels geeigneter Auswertungsalgorithmen kann dann auf die Topographie des Patientenkörpers 3 geschlossen werden und diese beispielsweise mit einer Referenztopographie verglichen werden. Durch eine entsprechende Ansteuerung der Verstellantriebe der Unterlage 2 kann der Patient 3 in die gewünschte, durch die Referenztopographie vorgegebene Position bewegt werden. Auf diese Weise wird eine besonders genaue Positionierung des Patienten 3 auf der Lagerungshilfe 2 gewährleistet.

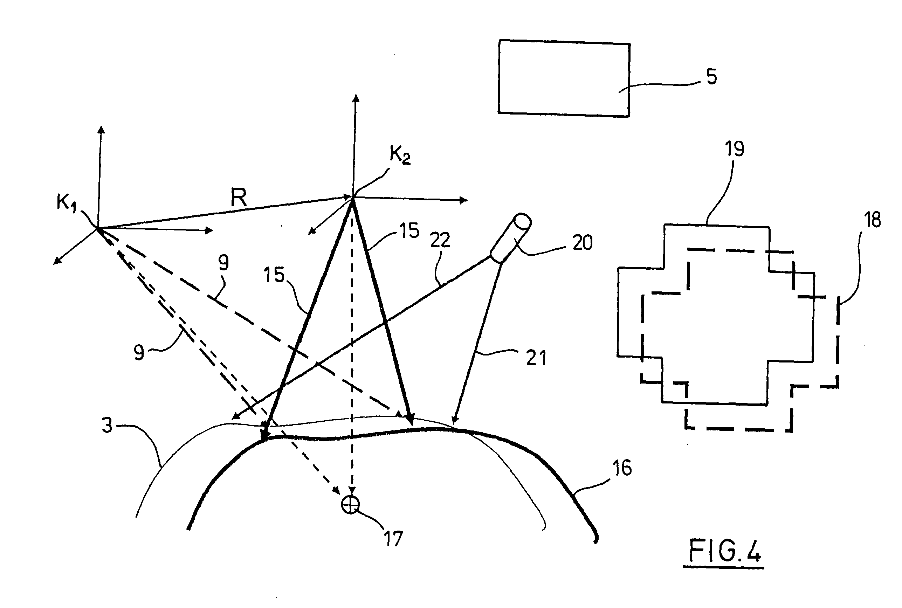

Die in Fig. 3 dargestellte Vorrichtung umfasst weiterhin ein Bestrahlungsgerät 14, vorliegend einen Linearbeschleuniger (LINAC). Das Bestrahlungsgerät 14 erzeugt eine ionisierende Strahlung zur Tumorbehandlung in dem Patientenkörper 3. Bei der Vorrichtung gemäß Fig. 3 werden wiederum von der Steuereinrichtung 5 zur Verfügung gestellte 3D-Koordinaten eines Sollschnittfeldes an die die entsprechende Körperregion erreichende Projektionseinrichtung 6 übermittelt. Die Projektionseinrichtung 6 projiziert das Sollschnittfeld mit Laserstrahlung 9 auf den Patientenkörper 3. Die Projektion des Sollschnittfelds erfolgt dabei wie bereits zu den Figuren 1 und 2 beschrieben. Gleichzeitig wird von dem Bestrahlungsgerät 14 eine der von dem Bestrahlungsgerät erzeugten ionisierenden Strahlung entsprechende Lichtstrahlung 15 erzeugt. Diese Lichtstrahlung 15 erzeugt auf dem Patientenkörper 3 ein Lichtschnittfeld, das dem Schnittfeld der von dem Bestrahlungsgerät 14 zu erzeugenden ionisierenden Strahlung mit der Patientenoberfläche entspricht. Anhand eines Vergleichs des von der Projektionseinrichtung 6 auf die Patientenkörperoberfläche projizierten Sollschnittfelds mit dem von dem Bestrahlungsgerät 14 erzeugten Lichtschnittfeld kann nun die korrekte Ausrichtung des Patienten kontrolliert werden. Dies soll anhand der schematischen Darstellung in Fig. 4 näher erläutert werden.

In Fig. 4 ist zu erkennen, dass für die Projektionseinrichtung 6 und das Bestrahlungsgerät 14 unterschiedliche Koordinatensysteme K1, K2, gültig sind. Ihre Koordinatenursprünge sind über einen Transformationsvektor R ineinander überführbar. Der reale Patientenkörper 3 ist in dem in Fig. 4 dargestellten Beispiel von einer Sollposition 16 des Patientenkörpers 3 entfernt. Das Isozentrum für die Bestrahlung ist für die Sollposition 16 des Patientenkörpers 3 bei 17 gezeigt. Aufgrund der Abweichung des realen Patientenkörpers 3 von der Sollposition 16 sind das von der Projektionseinrichtung 6 auf den Patientenkörper 3 projizierte Sollschnittfeld 18 und das von dem Bestrahlungsgerät 14 auf die Patientenkörperoberfläche gestrahlte Lichtschnittfeld 19 nicht deckungsgleich, wie in Fig. 4 schematisch gezeigt. Die Vorrichtung weist nun weiterhin eine Sensoreinrichtung 20, vorliegend eine Kamera 20, auf. Das Gesichtsfeld der Kamera 20 wird durch die Pfeile 21, 22 schematisch dargestellt. Die Kamera 20 nimmt somit sowohl das Sollschnittfeld 18 als auch das Lichtschnittfeld 19 auf. Diese aufgenommenen Daten werden von der Kamera 20 über nicht dargestellte Leitungen an die in die Steuereinrichtung 5 integrierte Auswerteeinrichtung geleitet. Mittels geeigneter Bildauswertealgorithmen, wie sie an sich bekannt sind, wird von der Auswerteeinrichtung eine Abweichung zwischen dem Sollschnittfeld 18 und dem Lichtschnittfeld 19 ermittelt. Insbesondere berechnet die Auswerteeinrichtung eine für eine Deckungsgleichheit zwischen dem Sollschnittfeld 18 und dem Lichtschnittfeld 19 erforderliche Position der Lagerungshilfe 2 des Patientenkörpers 3. Anschließend werden die Verstellantriebe der Lagerungshilfe 2 von der in die Steuereinrichtung 5 integrierten Auswerteeinrichtung so angesteuert, dass die Lagerungshilfe 2 in die erforderliche Position bewegt wird, so dass das Sollschnittfeld 18 und das Lichtschnittfeld 19 deckungsgleich sind. Der Patient befindet sich nun in der gewünschten Position und die Bestrahlung mit dem Bestrahlungsgerät 14 kann beginnen.

Selbstverständlich kann die Vorrichtung weiterhin ein Echtzeit- oder Nahechtzeit-Oberflächenerfassungssystem aufweisen, um die Projektion des Sollschnittfelds beispielsweise abhängig von einer durch die Atmung des Patienten veränderlichen Oberfläche des Patientenkörpers 3 anzupassen.

## Patentansprüche

1. Vorrichtung zur Darstellung eines Feldes auf der dreidimensionalen Oberfläche eines Patientenkörpers, mit einer Steuereinrichtung, die dreidimensionale Koordinaten mindestens eines auf der Oberfläche des Patientenkörpers darzustellenden Feldes bereitstellt, wobei das Feld ein Sollschnittfeld eines Strahlenfeldes auf der Oberfläche des Patientenkörpers vorgibt,
**dadurch gekennzeichnet, dass** mindestens eine mindestens einen Laser aufweisende Projektionseinrichtung (6) vorgesehen ist, mit der anhand der bereitgestellten Koordinaten das Sollschnittfeld (18) auf die dreidimensionale Oberfläche des Patientenkörpers (3) projizierbar ist, indem mindestens ein von dem Laser erzeugter Laserstrahl (9) ausreichend schnell entlang der Kontur des Sollschnittfelds (18) geführt werden kann, so dass der Eindruck einer geschlossenen Kontur um das Sollschnittfeld (18) entsteht.

2. Vorrichtung nach Anspruch 1, **dadurch gekennzeichnet, daß** die Projektionseinrichtung (6) mindestens zwei drehbare Spiegel aufweist, mit denen der Laserstrahl (9) auf die Oberfläche des Patientenkörpers (3) reflektiert und entlang der Kontur des Sollschnittfelds (18) geführt werden kann.

3. Vorrichtung nach einem der Ansprüche 1 oder 2, **dadurch gekennzeichnet, dass** mittels der Steuereinrichtung (5) eine Kalibrierung der Projektionseinrichtung (6) durchführbar ist, indem mittels der Steuereinrichtung (5) Kalibrierkoordinaten zur Verfügung gestellt und ein von der Projektionseinrichtung (6) anhand der bereitgestellten Kalibrierkoordinaten auf eine Oberfläche projizierter Kalibrierpunkt oder ein projiziertes Kalibrierfeld mit einem Sollkalibrierpunkt bzw. einem Sollkalibrierfeld vergleichbar ist.

4. Vorrichtung nach einem der vorhergehenden Ansprüche, **dadurch gekennzeichnet, dass** sie ein Bestrahlungsgerät (14) zum Erzeugen einer ionisierenden Strahlung für die Strahlentherapie aufweist, wobei mit dem Bestrahlungsgerät (14) eine der von dem Bestrahlungsgerät (14) erzeugten Strahlung entsprechende Lichtstrahlung (15) erzeugbar ist und, dass die Vorrichtung (1) eine Sensoreinrichtung (20) aufweist, mit der das Sollschnittfeld (18) und ein Lichtschnittfeld (19) der Lichtstrahlung (15) des Bestrahlungsgeräts (14) auf der Oberfläche des Patientenkörpers (3) aufgenommen werden können.

5. Vorrichtung nach Anspruch 4, **dadurch gekennzeichnet, dass** eine Auswerteeinrichtung vorgesehen ist, die dazu ausgebildet ist, eine Abweichung zwischen dem Sollschnittfeld (18) und dem Lichtschnittfeld (19) zu ermitteln.

6. Vorrichtung nach einem der vorhergehenden Ansprüche, **dadurch gekennzeichnet, dass** sie eine den Patientenkörper (3) aufnehmende, mittels geeigneter Verstellantriebe entlang mindestens dreier Freiheitsgrade verstellbar gelagerte Lagerungshilfe (2) aufweist.

7. Vorrichtung nach den Ansprüchen 5 und 6, **dadurch gekennzeichnet, dass** die Auswerteeinrichtung dazu ausgebildet ist, eine für eine Deckungsgleichheit zwischen dem Sollschnittfeld (18) und dem Lichtschnittfeld (19) erforderliche Position der Lagerungshilfe (2) zu ermitteln.

8. Vorrichtung nach Anspruch 7, **dadurch gekennzeichnet, dass** die Auswerteeinrichtung dazu ausgebildet ist, die Verstellantriebe so anzusteuern, dass die Lagerungshilfe (2) in die erforderliche Position bewegt wird.

9. Vorrichtung nach Anspruch 6 oder nach Anspruch 6 und einem der vorhergehenden Ansprüche, **dadurch gekennzeichnet, dass** eine Einrichtung (13) zur Ermittlung der Topographie eines auf der Lagerungshilfe (2) befindlichen Patientenkörpers (3) vorgesehen ist, die mindestens einen Laserscanner aufweist, mit dem eine Laserlinie auf den Patientenkörper (3) gerichtet werden kann, und mindestens eine Aufnahmeeinrichtung, mit der die von dem Patientenkörper (3) reflektierte Laserlinie aufgenommen werden kann.

10. Vorrichtung nach Anspruch 9, **dadurch gekennzeichnet, dass** eine Topographieauswerteeinrichtung vorgesehen ist, die dazu ausgebildet ist, aus den von der Aufnahmeeinrichtung aufgenommen Daten die Topographie des Patientenkörpers (3) zu ermitteln, mit einer Solltopographie zu vergleichen und anhand des Vergleichs eine für eine Deckungsgleichheit zwischen der gemessenen Topographie und der Solltopographie erforderliche Position der Lagerungshilfe (2) zu ermitteln.

11. Vorrichtung nach einem der vorhergehenden Ansprüche, **dadurch gekennzeichnet, dass** die Projektionseinrichtung (6) entlang einer im Wesentlichen kreisförmigen Bahn (7) um eine den Patientenkörper (3) aufnehmende Lagerungshilfe (2) bewegbar angeordnet ist.

12. Vorrichtung nach einem der vorhergehenden Ansprüche, **dadurch gekennzeichnet, dass** sie eine Einrichtung zur Echtzeit- oder Nah-Echtzeitermittlung einer dreidimensionalen Oberflächenform eines auf einer Lagerungshilfe (2) befindlichen Patientenkörpers (3) aufweist, wobei von der Einrichtung ermittelte dreidimensionale Oberflächendaten in Echtzeit oder Nah-Echtzeit an die Steuereinrichtung (5) übermittelbar sind.

13. Vorrichtung nach Anspruch 12, **dadurch gekennzeichnet, dass** die Einrichtung zur Echtzeit- oder Nah-Echtzeitermittlung der Oberflächenform des Patientenkörpers (3) mindestens einen Laser aufweist, mit dem mindestens eine Laserlinie auf den Patientenkörper (3) gerichtet werden kann, und mindestens einen Matrixsensor, mit dem die von dem Patientenkörper (3) reflektierte Laserlinie detektiert werden kann.

14. Vorrichtung nach Anspruch 12 oder 13, **dadurch gekennzeichnet, dass** die Steuereinrichtung (5) dazu ausgebildet ist, die Projektionseinrichtung (6) so anzusteuern, dass diese das Sollschnittfeld (18) nur bei einer vorgegebenen Oberflächenposition des Patientenkörpers (3) auf die dreidimensionale Oberfläche des Patientenkörpers (3) projiziert.

15. Vorrichtung nach einem der Ansprüche 12 bis 14, **dadurch gekennzeichnet, dass** die Steuereinrichtung (5) dazu ausgebildet ist, die der Projektionseinrichtung (6) zur Verfügung gestellten Koordinaten des Sollschnittfelds (18) in Abhängigkeit von der jeweiligen Oberfläche des Patientenkörpers (3) in Echtzeit oder Nah-Echtzeit so anzupassen, dass das projizierte Schnittfeld (18) zu jeder Zeit das Sollschnittfeld (18) des für die Strahlentherapie vorgesehenen Therapiefelds mit der Oberfläche des Patientenkörpers (3) darstellt.

16. Verfahren zur Darstellung eines Feldes auf der dreidimensionalen Oberfläche eines Patientenkörpers, bei dem von einer Steuereinrichtung dreidimensionale Koordinaten mindestens eines auf der Oberfläche des Patientenkörpers darzustellenden Feldes bereitgestellt werden, wobei das Feld ein Sollschnittfeld eines Strahlenfeldes auf der Oberfläche des Patientenkörpers vorgibt,
**dadurch gekennzeichnet, dass** mit mindestens einer mindestens einen Laser aufweisenden Projektionseinrichtung (6) anhand der bereitgestellten Koordinaten das Sollschnittfeld (18) auf die dreidimensionale Oberfläche des Patientenkörpers (3) projiziert wird, indem mindestens ein von dem Laser erzeugter Laserstrahl (9) ausreichend schnell entlang der Kontur des Sollschnittfelds (18) geführt wird, so dass der Eindruck einer geschlossenen Kontur um das Sollschnittfeld (18) entsteht.

17. Verfahren nach Anspruch 16**, dadurch gekennzeichnet, daß** der Laserstrahl (9) durch mindestens zwei drehbare Spiegel auf die Oberfläche des Patientenkörpers (3) reflektiert und entlang der Kontur des Sollschnittfelds (18) geführt wird.

18. Verfahren nach einem der Ansprüche 16 oder 17, **dadurch gekennzeichnet, dass** eine Kalibrierung der Projektionseinrichtung (6) durchgeführt wird, indem Kalibrierkoordinaten zur Verfügung gestellt und ein von der Projektionseinrichtung (6) anhand der bereitgestellten Kalibrierkoordinaten auf eine Oberfläche projizierter Kalibrierpunkt oder ein projiziertes Kalibrierfeld mit einem Sollkalibrierpunkt bzw. einem Sollkalibrierfeld verglichen wird.

19. Verfahren nach einem der Ansprüche 16 bis 18, **dadurch gekennzeichnet, dass** mit einem Bestrahlungsgerät (14) zum Erzeugen einer ionisierenden Strahlung für die Strahlentherapie eine der von dem Bestrahlungsgerät (14) erzeugten Strahlung entsprechende Lichtstrahlung (15) erzeugt wird und, dass das Sollschnittfeld (18) und ein Lichtschnittfeld (19) der Lichtstrahlung (15) des Bestrahlungsgeräts (14) auf der Oberfläche des Patientenkörpers (3) aufgenommen wird.

20. Verfahren nach Anspruch 19, **dadurch gekennzeichnet, dass** eine Abweichung zwischen dem Sollschnittfeld (18) und dem Lichtschnittfeld (19) ermittelt wird.

21. Verfahren nach Anspruch 20, **dadurch gekennzeichnet, dass** eine für eine Deckungsgleichheit zwischen dem Sollschnittfeld (18) und dem Lichtschnittfeld (19) erforderliche Position einer den Patientenkörper (3) aufnehmenden, verstellbar gelagerten Lagerungshilfe (2) ermittelt wird.

22. Verfahren nach Anspruch 21, **dadurch gekennzeichnet, dass** die Lagerungshilfe (2) in die erforderliche Position bewegt wird.

23. Verfahren nach einem der Ansprüche 16 bis 22, **dadurch gekennzeichnet, dass** zur Ermittlung der Topographie eines auf einer verstellbar gelagerten Lagerungshilfe (2) befindlichen Patientenkörpers (3) eine Laserlinie auf den Patientenkörper (3) gerichtet wird und, dass die von dem Patientenkörper reflektierte Laserlinie aufgenommen wird.

24. Verfahren nach Anspruch 23, **dadurch gekennzeichnet, dass** aus den aufgenommen Daten die Topographie des Patientenkörpers (3) ermittelt, mit einer Solltopographie verglichen und anhand des Vergleichs eine für eine Deckungsgleichheit zwischen der gemessenen Topographie und der Solltopographie erforderliche Position der Lagerungshilfe (2) ermittelt wird.

25. Verfahren nach einem der Ansprüche 16 bis 24, **dadurch gekennzeichnet, dass** die dreidimensionale Oberflächenform eines Patientenkörpers (3) in Echtzeit- oder Nah-Echtzeit ermittelt wird und die ermittelten dreidimensionalen Oberflächendaten in Echtzeit oder Nah-Echtzeit an die Steuereinrichtung (5) übermittelt werden.

26. Verfahren nach Anspruch 25, **dadurch gekennzeichnet, dass** zur Echtzeit- oder Nah-Echtzeitermittlung der Oberflächenform des Patientenkörpers (3) mindestens eine Laserlinie auf den Patientenkörper (3) gerichtet wird, und mit mindestens einem Matrixsensor die von dem Patientenkörper (3) reflektierte Laserlinie detektiert wird.

27. Verfahren nach Anspruch 25 oder 26, **dadurch gekennzeichnet, dass** die Projektionseinrichtung (6) so angesteuert wird, dass diese das Sollschnittfeld (18) nur bei einer vorgegebenen Oberflächenposition des Patientenkörpers (3) auf die dreidimensionale Oberfläche des Patientenkörpers (3) projiziert.

28. Verfahren nach einem der Ansprüche 25 bis 27, **dadurch gekennzeichnet, dass** die Steuereinrichtung (5) die der Projektionseinrichtung (6) zur Verfügung gestellten Koordinaten des Sollschnittfelds (18) in Abhängigkeit von der jeweiligen Oberfläche des Patientenkörpers (3) in Echtzeit oder Nah-Echtzeit so anpasst, dass das projizierte Schnittfeld (18) zu jeder Zeit das Sollschnittfeld (18) des für die Strahlentherapie vorgesehenen Therapiefelds mit der Oberfläche des Patientenkörpers (3) darstellt.
